# EUROPEAN PATENT APPLICATION

(11) **EP 4 218 673 A1**
(43) Date of publication of application: **02.08.2023**
(21) Application number: 22837074.8
(22) Date of filing: 19.08.2022
(51) Int. Cl.: A61F 2/24

(54) **IMPLANT DELIVERY HANDLE, AND DELIVERY SYSTEM AND OPERATING METHOD THEREFOR**

(30) Priority: 12.10.2021 CN 202111189005
(71) Applicant: Jiangsu Trulive Medtech Co., Ltd, Rudong County Nantong Jiangsu 226400 (CN)
(72) Inventor: SUN, Yunyan, Nantong, Jiangsu 226400 (CN); ZHAO, Jing, Nantong, Jiangsu 226400 (CN); ZHANG, Wei, Nantong, Jiangsu 226400 (CN); WEN, Jing, Nantong, Jiangsu 226400 (CN)
(74) Representative: Nordmeyer, Philipp Werner
(86) International application number: PCT/CN2022/113496
(87) International publication number: WO 2023/280329

(57) **Abstract**

An implant delivery handle, a delivery system and a method of operation thereof are disclosed. The implant delivery handle is able to switch an inner catheter (1) between unlocked and locked configurations through pivoting a locking knob (43) about an axis of the inner catheter (1) to cause a locking part (42) to exert or withdraw a radial pressure on the inner catheter (1). The implant delivery handle has a simplified structure and can be operated more easily. Moreover, the locking knob (43) for the inner catheter (1) and the control handwheel (33) for the flexible catheter (2) employ distinguishable designs, which effectively prevent a surgeon from being confused with the two actions. Instead of locking/unlocking the catheter through circumferentially rotating a rotary wheel as is conventional, the inner catheter (1) can be switched between the locked and unlocked configurations through compressing or uncompressing a collet (421) in a locking part (42), wherein the collet has a slot (4211) axially extending therethrough.

## Description

### TECHNICAL FIELD

The present invention relates to the field of medical devices and, in particular, to an implant delivery handle, a delivery system and a method of operation thereof.

### BACKGROUND

Heart valves are flap-like structures in the organs of humans and some animals, which are able to open and close. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent the backward flow of blood.

With the development of social economy and the aging of population, the incidence of valvular heart disease has increased significantly. Studies have shown that this figure has reached up to 13.3% among those 75 years or older. At present, traditional surgical treatment remains first choice for patients with severe valvular disease. However, for those with advanced ages, complications in multiple organs, a history of thoracotomy or poor heart functions, the traditional surgical approach is associated with high risk and high mortality or even precludes some patients.

Interventional valve implantation is a brand new minimally invasive valve replacement technique that has been developed abroad in recent years, which involves loading a valve prosthesis in a delivery system and delivering it into the body of a patient through a catheter. The prosthesis can functionally replace the patient's dysfunctional native valve and improve his/her cardiac condition. This technique is able to treat aortic valve disease without surgically opening the chest or stopping the heartbeat, which may cause significant trauma to the patient.

Valve surgery is highly challenging and requires a surgeon to be highly focused to perform a series of actions involving controllable flexibility, displacement and locking required by delivery of, for example, an artificial aortic valve for use in a transfemoral aortic valve replacement procedure. Most traditional delivery handles for this purpose utilize threaded transmission and are thus equipped with many circumferential rotary components, and a surgeon must be accurately aware of the functions of these individual components during surgery. For valve surgery procedures which are labor-intensive, highly risky and prone to frequent emergency situations, this tends to lead to operational mistakes or errors, which may lead to surgical failure in severe cases.

### SUMMARY OF THE INVENTION

It is an object of the present invention to provide an implant delivery handle, a delivery system and a method of operation thereof, which can solve the above-described problems of too many components and tedious operations.

To this end, the present invention provides an implant delivery handle, which comprises a locking handle disposed along an axial direction of a catheter assembly. The locking handle comprises a locking part and a locking knob. The locking part is sleeved over an inner catheter in the catheter assembly so as to be sandwiched between the locking knob and the inner catheter. The locking knob is configured to pivot about an axis of the inner catheter to apply a radial pressure to the inner catheter through the locking part, thereby switching the inner catheter between an unlocked configuration and a locked configuration.

The locking knob comprises a collet sleeved over the inner catheter. The locking knob is abutment against the collet. The collet has an inner diameter greater than an outer diameter of the inner catheter. The collet has a slot axially extending therethrough. In case of the collet being subject to a radial pressure, a circumferential length of the slot decreases, resulting in a reduced inner diameter of the collet, wherein the reduced inner diameter of the collet is smaller than the outer diameter of the inner catheter. The collet locks the inner catheter through contraction of the collet.

Optionally, the implant delivery handle may comprise a control handle disposed along the axial direction of a catheter assembly at a distal end of the locking handle. The control handle may be coupled to a flexible catheter in the catheter assembly.

Additionally, the control handle may comprise a control handwheel coupled to a control wire on the flexible catheter and configured to rotate to exert a force on the control wire to provide bending control to the catheter assembly.

Optionally, the locking knob may further comprise a collet block sleeved over the collet and be configured to abut against the collet via the collet block. A surface of the collet block facing the collet matches the outer surface of the collet in shape, and a surface of the collet block facing the locking knob is flat.

Further, the locking handle may further comprise a locking center piece and a locking shell. The locking center piece may be secured within the locking shell. The locking knob may extend through a sidewall of the locking shell and be threadedly coupled to a sidewall of the locking center piece.

The locking center piece may comprise an axial bore axially extending therethrough. The inner catheter and the collet are disposed in the axial bore, and a proximal end of the axial bore is provided with a mating groove and a mating locating protrusion that are circumferentially arranged, the mating groove is configured for fitting of the collet block and the mating locating protrusion is received in the slot.

Further, the locking knob may comprise a connecting part and a dial part. The connecting part may extend through the side wall of the locking shell and be threadedly coupled to the sidewall of the locking center piece and configured to abut against the collet block.

The dial part may comprise a manipulation piece sleeved over an end portion of the connecting part away from the collet block and threadedly fastened to the connecting part. The manipulation piece may be disposed facing a proximal end of the implant delivery handle and is configured to drive, at a proximal end of the locking handle, the connecting part to pivot about the axis of the inner catheter.

Optionally, the control handle may further comprise:
a control shell comprising an inner lumen, wherein the inner lumen comprises a distal first portion and a proximal second portion, wherein an inner diameter of the distal first portion is smaller than an inner diameter of the proximal second portion;
a control center piece, inserted in the inner lumen, wherein the control handwheel is sleeved over the control center piece;
a proximal snap part disposed in the first portion;
a distal snap part disposed in the first portion and located at a distal end of the proximal snap part, wherein the distal snap part has an outer diameter greater than an inner diameter of the first portion; and
a slider threadedly coupled to the control handwheel and located at a proximal end of the proximal snap part, wherein the slider is able to move axially between the proximal snap part and a proximal end portion of the control handwheel.

Further, the control center piece may comprise a tubular first part and a tubular second part sleeved over a proximal end portion of the first part, wherein a flexible catheter is secured in the first part, and wherein the control handwheel is sleeved over the first part.

The first part may be provided in an outer circumference thereof with a proximal snap slot and a distal snap slot, which are axially spaced apart from each other. The proximal snap slot may be provided at a distal end of the control handwheel and configured to secure the proximal snap part. The distal snap slot may be configured to secure the distal snap part.

The first part may be further provided in the outer circumference thereof with a wire slot, which extends through a sidewall of the first part and has the same axial direction as the first part. The wire slot may be located between a distal end face of the first part and the proximal snap slot. The wire slot may be configured so that a control wire in the flexible catheter leads out the tubular body of the first part and is tied to the slider.

Further, the control handwheel may comprise a proximal portion and a distal portion, which are joined to each other. The proximal portion may have an outer diameter greater than an outer diameter of the distal portion, and a distal end face of the proximal portion comprises a receptacle having a same axial direction as the first part. A diameter of a side wall of the receptacle proximal to an axis is equal to the outer diameter of the distal portion.

The slider may be threadedly coupled to an outer wall of the distal portion in the receptacle. The slider is able to move axially on the control handwheel between the proximal snap part and a bottom surface of the receptacle.

In a second aspect, the present invention also provides an implant delivery system comprising the implant delivery handle as defined above. The implant delivery system further comprises a catheter assembly and a Y-shaped catheter holder. The Y-shaped catheter holder is disposed at the proximal end of the implant delivery handle, and the catheter assembly comprises an inner catheter and a flexible catheter sleeved over the inner catheter. A proximal end of the inner catheter passes through the implant delivery handle and is secured to the Y-shaped catheter holder. The flexible catheter is secured to the control handle of the implant delivery handle. The control handle is configured to enable the flexible catheter to provide bending control, and the locking handle of the implant delivery handle is configured to switch the inner catheter between locked and unlocked configurations of axial displacement thereof.

In a third aspect, the present invention also provides a method of operating the implant delivery system as defined above, which comprises:
rotating the control handwheel to cause the control wire to bend as a result of a force exerted by the slider on the control wire increasing, or to cause a distal end of the control wire to return to an original configuration as a result of a force exerted by the slider on the control wire decreasing; and
pivoting the dial part to a first position where the locking part does not exert a radial pressure on the collet and the inner catheter is in the unlocked configuration in which the inner catheter is capable of axial displacement, pivoting the dial part to a a second position where the locking part exerts a radial pressure on the collet to reduce its inner diameter and the inner catheter is in the locked configuration in which it is incapable of axial displacement.

The present invention has the following benefits over the prior art:
The implant delivery handle, delivery system and method provided in the present invention are able to switch an inner catheter between unlocked and locked configurations through pivoting the locking knob about an axis of the inner catheter to cause a locking part to exert or withdraw a radial pressure on the inner catheter. The implant delivery handle has a simplified structure and can be operated more easily. Moreover, the locking knob for the inner catheter and the control handwheel for the flexible catheter employ distinguishable designs, which effectively prevent a surgeon from being confused with the two actions. Instead of locking/unlocking the catheter through circumferentially rotating a rotary wheel as is conventional, the inner catheter can be switched between the locked and unlocked configurations through compressing or uncompressing a collet in a locking knob, which has a slot axially extending through.

Moreover, the implant delivery handle can provide bending control to the catheter assembly by rotating the control handwheel to exert a force on the control wire.

### BRIEF DESCRIPTION OF THE DRAWINGS

Figs. 1a to 1c are schematic illustrations of an implant delivery system according to an embodiment of the present invention.
Fig. 2 is a schematic exploded view of a control handle according to an embodiment of the present invention.
Fig. 3 is a schematic illustration of a control center piece according to an embodiment of the present invention.
Figs. 4a to 4b are schematic illustrations of a control handwheel according to an embodiment of the present invention.
Figs. 5a to 5b are schematic illustrations of a control shell according to an embodiment of the present invention.
Fig. 6 is a schematic cross-sectional view of a part of a locking handle according to an embodiment of the present invention.
Figs. 7a to 7b schematically illustrate a locking part according to an embodiment of the present invention.
Fig. 8 is a schematic illustration of a locking center piece according to an embodiment of the present invention.
Figs. 9a to 9c schematically illustrate a locking knob according to an embodiment of the present invention.
Figs. 10a to 10b are schematic illustrations of a locking handle in unlocked and locked configurations of a catheter according to an embodiment of the present invention.
Figs. 11a to 11c schematically illustrate a conventional locking mechanism for an inner catheter.

In these figures,
1, an inner catheter; 11, a force application area for the inner catheter;
2, a flexible catheter;
3, a control handle; 31, a control center piece; 311, a first part; 3111, a first through hole; 3112, a distal snap slot; 3113, a proximal snap slot; 3114, a first wedge surface; 3115, a wire slot; 312, a second part; 3121, a vent hole; 32, a slider; 33, a control handwheel; 331, a distal portion; 332, a proximal portion; 3321, a receptacle; 3322, an arch-shaped structure; 333, a central bore; 34, a proximal snap part; 35, a distal snap part; 36, a control shell; 361, an annular recess; 362, a first portion; 363, a second portion; 364, a groove; 37, a stress-dispersing tube;
4, a locking handle; 41, a locking center piece; 411, a distal end structure; 4111, a first locating groove; 412, a proximal end structure; 4121, a second through hole; 413, an axial bore; 4131, a mating locating protrusion; 4132, a mating groove; 42, a locking part; 421, a collet; 4211, a slot; 422, a collet block; 4221, a flat surface; 4222, an arc-shaped surface; 43, a locking knob; 431, a connecting part; 4311, a hexagonal boss; 4312, a threaded shaft; 4313, a threaded blind hole; 432, a dial part; 4321, a manipulation piece; 4322, a raised annulus; 4323, a second locating groove; 44, a locking shell;
5, a Y-shaped catheter holder;
6, an implant; A, a first position; B, a second position; I, a first direction;
7, a conventional catheter locking mechanism; 71, a conventional collet block; 711, a chamfer; 72, a collet; 721, an axial slot; 722, a distal bevel; and 723, a compressible section.

### DETAILED DESCRIPTION

The proposed implant delivery handle, delivery system and method of operation thereof will be described in greater detail below. The present invention will be described in greater detail below with reference to the accompanying drawings, which present preferred embodiments of the invention. It would be appreciated that those skilled in the art can make changes to the invention disclosed herein while still obtaining the beneficial results thereof. Therefore, the following description shall be construed as being intended to be widely known by those skilled in the art rather than as limiting the invention.

For the sake of clarity, not all features of actual implementations are described. In the following, description and details of well-known functions and structures are omitted to avoid unnecessarily obscuring the invention. It should be appreciated that in the development of any such actual implementation, numerous implementation-specific decisions must be made to achieve specific goals of the developers, such as compliance with system-related and business-related constrains, which may vary from one implementation to another. Moreover, it should be appreciated that such a development effort might be complex and time consuming, but would nevertheless be a routine undertaking for those of ordinary skill in the art.

Objects and features of the present invention will become more apparent upon reading the following more detailed description thereof made with reference to the accompanying drawings and particular embodiments. Note that the figures are provided in a very simplified form not necessarily drawn to exact scale and for the only purpose of facilitating easy and clear description of the disclosed embodiments. As used herein, the term "or" is generally employed in the sense of "and/or", unless the context clearly dictates otherwise. As used herein, the terms "inner", "outer" and similar terms are merely illustrative and do not represent the only implementation possible. As used herein, the terms "proximal" and "distal" are employed to describe relative orientations, relative positions and directions between components of a medical device or actions thereof, as viewed by a physician operating the device. Without wishing to be limiting, a "proximal end" usually refers to an end closer to the operator, and a "distal end" usually refers to an end closer to the heart of a patient, during normal operation of the medical device.

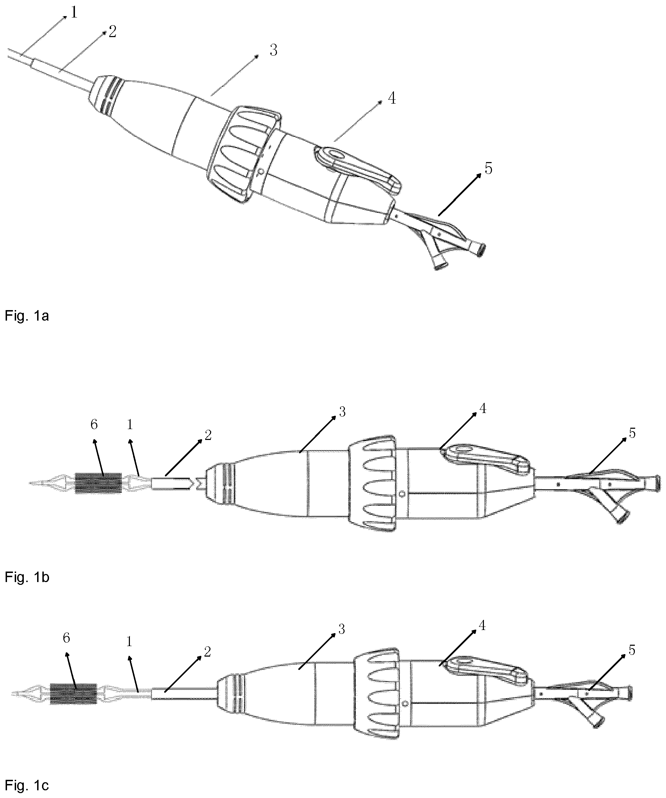

Fig. 1a is a schematic illustration of an implant delivery handle according to an embodiment of the present invention. Fig. 1b is a schematic illustration of the implant delivery system according to the embodiment in a configuration prior to an implantation process. Fig. 1c is a schematic illustration of the implant delivery system according to the embodiment in a configuration suitable for balloon pacing. As shown in Figs. 1a to 1c, the implant delivery system according to this embodiment includes an implant delivery handle, a catheter assembly and a Y-shaped catheter holder 5. The Y-shaped catheter holder 5 is disposed at a proximal end of the implant delivery handle.

The catheter assembly includes an inner catheter 1 and a flexible catheter 2 sleeved over the inner catheter 1. A distal end of the inner catheter 1 protrudes beyond a distal end of the flexible catheter 2 and is configured for the engagement of an implant 6 (e.g., a prosthetic valve) thereat. The implant delivery handle includes a distal control handle 3 and a proximal locking handle 4. The flexible catheter 2 is proximally inserted in and secured to the implant delivery handle and configured to enable bending control of the catheter assembly by the implant delivery handle. The inner catheter 1 is proximally inserted successively through the control handle 3 and the locking handle 4 and secured to the Y-shaped catheter holder 5. The locking handle 4 is configured to circumferentially rotate to switch the inner catheter 1 between locked and unlocked configurations. In the unlocked configuration, axial displacement of the inner catheter 1 relative to the implant delivery handle and the flexible catheter 2 is allowed.

A control wire is passed through the flexible catheter 2 and proximal ends of the control wire and the flexible catheter 2 are coupled to the control handle 3. Specifically, the proximal end of the flexible catheter 2 may be secured to the control handle 3 by an adhesive or otherwise, and the proximal end of the control wire is secured to the control handle 3 so as to allow the control handle 3 to provide the control wire with an axial force required by bending control of the catheter assembly.

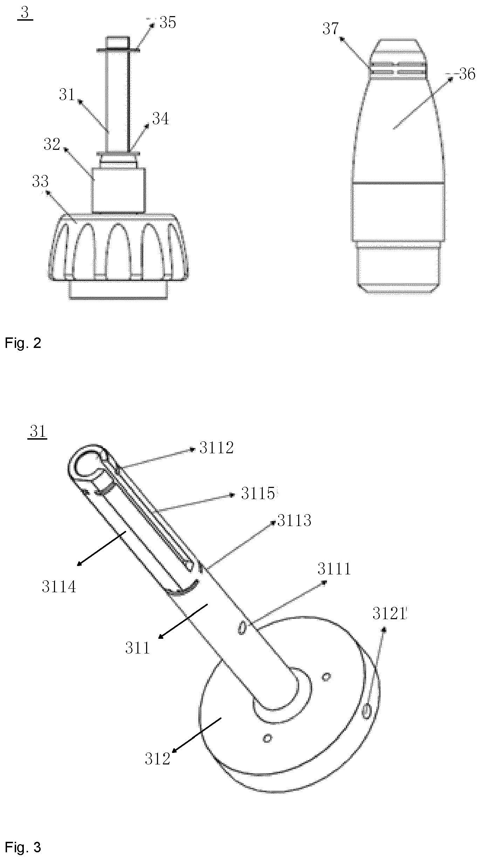

Fig. 2 is a schematic exploded view of the control handle according to an embodiment of the present invention. As shown in Fig. 2, the control handle 3 includes a control shell 36, a control center piece 31, a slider 32, a control handwheel 33, a proximal snap part 34 and a distal snap part 35. The slider 32 is disposed over the control handwheel 33, and the distal snap part 35, the proximal snap part 34 and the control handwheel 33 are sleeved over the control center piece 31 sequentially from the distal end to the proximal end. The control center piece 31 is inserted in the control shell 36, a proximal end portion of the control center piece 31 and the control handwheel 33 are exposed at a proximal end of the control shell 36, while the slider 32, the proximal snap part 34, the distal snap part 35 and a distal end portion of the control center piece 31 are all housed in the control shell 36. The control wire is secured to the slider 32 by mechanical fastening, gluing, welding or otherwise.

Fig. 3 is a schematic illustration of the control center piece according to an embodiment of the present invention. As shown in Fig. 3, the control center piece 31 includes a tubular first part 311 and a tubular second part 312 sleeved over a proximal end portion of the first part 311. A proximal end portion of the flexible catheter 2 is secured within the first part 311. A first through hole 3111 is provided in a wall of the first part 311 and is located above a wall of the flexible catheter 2. Glue may be introduced through the first through hole 3111 into a clearance between the first part 311 and the flexible catheter 2, to adhesively secure the flexible catheter 2 within the tubular body of the first part 311. Specifically, when the glue is UV-curable, the first part 311 may be made of a transparent material which allows UV radiation to transmit through the tubular wall of the first part 311 to irradiate and cause curing of the glue.

In an outer circumference of the first part 311, a proximal snap slot 3113 and a distal snap slot 3112 that are axially spaced apart from each other are provided. The proximal snap slot 3113 is provided at a proximal end of the distal snap slot 3112, and both the proximal snap slot 3113 and the distal snap slot 3112 are provided at distal ends of the slider 32 and the control handwheel 33. The proximal snap part 34 is fastened in the proximal snap slot 3113, and the distal snap part 35 in the distal snap slot 3112.

The outer circumference of a distal section of the first part 311 has a first wedge surface 3114 and a wire slot 3115 that are arranged circumferentially. The first wedge surface 3114 extends in an axial direction of the first part 311, and is located between a distal end face of the first part 311 and the proximal snap slot 3113, and is configured to cooperate with the control shell 36 to prevent rotation of the control center piece 31 relative to the control shell 36, so that the control center piece 31 is confined in a distal portion of the control shell 36 without circumferential displacement. That is, the control center piece 31 is totally fixed in position relative to the control shell 36.

The wire slot 3115 is provided in the wall of the first part 311, and an axial direction of the wire slot 3115 is as same as the axial direction of the first part 311. The control wire leads out of the first part 311 through the wire slot 3115, passes between two arc-shaped slots of the proximal snap slot 3113 and is then tied to the slider 32. This ensures that the control wire, when pulled, will not interfere with any mechanical component.

A vent hole 3121 is provided in a circumferential surface of the second part 312. The vent hole 3121 radially extends through both the first part 311 and the second part 312 and is configured to evacuate air from a space between the flexible catheter 2 and the inner catheter 1.

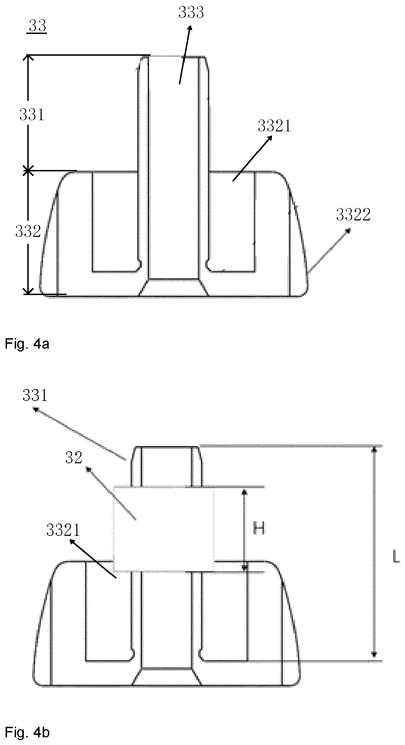

Figs. 4a to 4b are schematic illustrations of the control handwheel according to an embodiment of the present invention. Referring to Figs. 4a to 4b, in conjunction with Fig. 1a, the control handwheel 33 comprises a central bore 333 extending therethrough axially. The central bore 333 has a diameter greater than an outer diameter of the first part 311, and a proximal end of the central bore 333 comprises a tapered surface for guiding fast and accurate passage of the first part 311 through the control handwheel 33. The control handwheel 33 includes a proximal portion 332 and a distal portion 331, which are connected. The control handwheel 33 is disposed over the first part 311, with the proximal portion 332 being located at a distal end of the second part 312. The proximal portion 332 has an outer diameter, which is greater than an outer diameter of the distal portion 331 and is minimum at a distal end of the proximal portion 332. Moreover, the outer diameter of the proximal portion 332 is greater than an outer diameter of the second part 312.

The proximal portion 332 has a receptacle 3321 in its distal end face, which has an axial direction that is the same as the axial direction of the first part 311. The receptacle 3321 is a circular annular recess. The side wall of the receptacle 3321 close to an axis forms an inner annular and the side wall of the receptacle 3321 away from the axis forms an outer annular. The inner annular has a diameter that is equal to the outer diameter of the distal portion 331, and the outer annular has a diameter greater than both an outer diameter of the slider 32 and an outer diameter of a proximal end portion of the control shell 36. In this way, the slider 32 and the control shell 36 are axially displaceable in the receptacle 3321 so that upper limitation for bending control by the control handle 3 is provided by the proximal snap part 34 and that lower limitation for bending control by the control handle 3 is provided by a bottom surface of the receptacle 3321 (i.e., an inner surface of the receptacle 3321 proximal to the second part 312). Moreover, a stroke for bending control is defined by the difference between a distance L from the upper limitation to the lower limitation and an axial length H of the slider 32.

Preferably, external threads are provided on both the inner annular of the receptacle 3321 and an outer circumference of the distal portion 331, while the slider 32 is provided with an internal thread engageable with the external threads. In this way, the slider 32 can rotate to axially displace on the control handwheel 33 to be able to withstand a greater pulling force from the control wire or to be operated by the latter with a reduced force. The slider 32 and control handwheel 33 may be made of engineering plastics with high strength and small friction coefficients. Among others, polytetrafluoroethylene, polyetheretherketone and polyoxymethylene are preferred.

The proximal portion 332 is provided on its outer circumference with multiple arch-shaped structures 3322 each having the same axial direction as that of the first part 311. The arch-shaped structures 3322 may be either recessed or raised. Preferably, each arch-shaped structure 3322 has maximum cross-sectional area at a proximal end of the proximal portion 332.

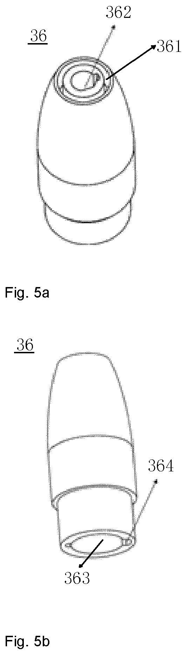

Figs. 5a to 5b are schematic illustrations of the control shell according to an embodiment of the present invention. Referring to Figs. 5a to 5b, in conjunction with Fig. 2, the control shell 36 comprises an annular recess 361 in a distal end face thereof, in which a stress-dispersing tube 37 is embedded. The stress-dispersing tube 37 is a tubular structure having a center axis coincident with a center axis of the control shell 36. The stress-dispersing tube 37 is made of rubber or silicone with a hardness lower than 60 HA. It serves to support the flexible catheter 2 and prevent its bending at a distal end of the implant delivery handle.

The control shell 36 comprises an inner lumen including a first portion 362 and a second portion 363. The first portion 362 is located at a distal portion of the inner lumen and is located at the inner side of an inner annular of the annular recess 361. The inner diameter of the first portion 362 is smaller than an outer diameter of the distal snap part 35, and the inner diameter of the second portion 363 is greater than the outer diameters of the slider 32, the distal snap part 35 and the proximal snap part 34. In this way, the slider 32 is also limited between the upper and lower limitations for bending control, i.e., between the distal snap part 35 and the proximal snap part 34. The first portion 362 has a second wedge surface for fitting the first wedge surface 3114. The second wedge surface can work with the first wedge surface 3114 to limit relative circumferential movement (i.e., rotation) of the control center piece 31 and the control shell 36.

An inner wall of the second portion 363 is provided with axial grooves 364, while the slider 32 is provided on its outer circumference with ridges that can fit in the grooves 364 to limit relative rotation of the slider 32 and the control shell 36. In this way, when the control handwheel 33 is rotated, the slider 32 can drive axial displacement of the control wire so as to achieve the bending control.

In order to assemble the handle, the flexible catheter 2 is fixed within the tubular body of the control center piece 31 by glue introduced through the first through hole 3111, and the slider 32 is sleeved over the control handwheel 33. Additionally, the control handwheel 33, the proximal snap part 34, the distal snap part 35 and the second part 312 are sleeved over the first part 311 of the control center piece 31 sequentially from the proximal end to the distal end. The proximal end of the control wire is passed out of the control center piece 31 through the wire slot 3115 and tied to the slider 32, and the slider 32 is threadedly engaged with the control handwheel 33. In this way, the proximal snap part 34, the distal snap part 35, the control center piece 31 and the control shell 36 are immobilized relative to one another, and rotation of the control handwheel 33 will cause axial displacement of the slider 32 within the range defined by the upper and lower limitations for bending control. Since the control handwheel 33, the proximal snap part 34, the distal snap part 35, the control center piece 31 and the control shell 36 do not move axially, the control wire tied to the slider will be axially pulled, thus accomplishing bending control of the catheter assembly.

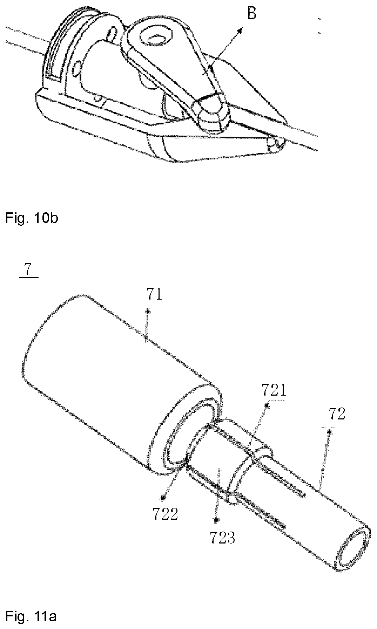

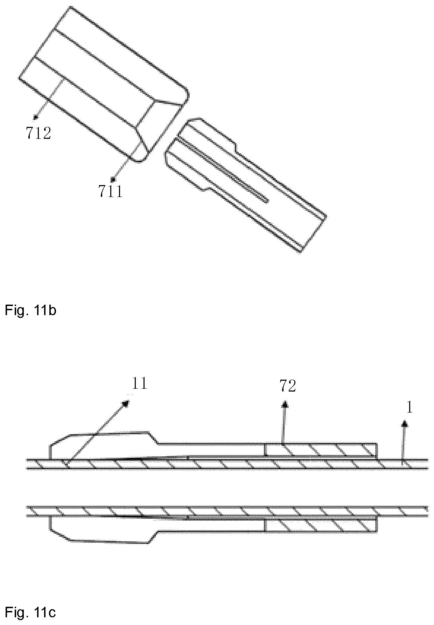

Figs. 11a to 11c schematically illustrate a conventional locking mechanism for an inner catheter 1. As shown in Figs. 11a to 11c, the conventional locking mechanism 7 includes a conventional collet block 71 and a collet 72, and the inner catheter 1 is disposed in the collet 72. The conventional collet block 71 has a chamfer 711, and the collet 72 has a distal bevel 722 that matches the chamfer 711. The collet 72 further includes circumferentially spaced axial slots 721 provided in a compressible section 723 of the collet 72. When the collet 72 is compressed by the conventional collet block 71, the axial slots 721 will be narrowed, reducing an inner diameter of the compressible section 723 and causing it to compress and lock the inner catheter 1. However, applying a pressure to the inner catheter 1 by compressing the distal portion of the collet 72 may cause irreversible damage to the inner catheter 1 or even other severe consequences such as fluid leakage due to a small force application area (a high pressure is produced at the location 11 of the catheter 1). In order to avoid this, a metal sleeve may be added to the location of the inner catheter 1 where it is locked to disperse the pressure. However, adding the metal sleeve to the catheter may lead to a material cost increase, and due to a limited allowable wall thickness of the catheter, this also means a reduced thickness of the polymer material, which may lead to fluid leakage at a high pressure. Therefore, this is very challenging in terms of design and fabrication.

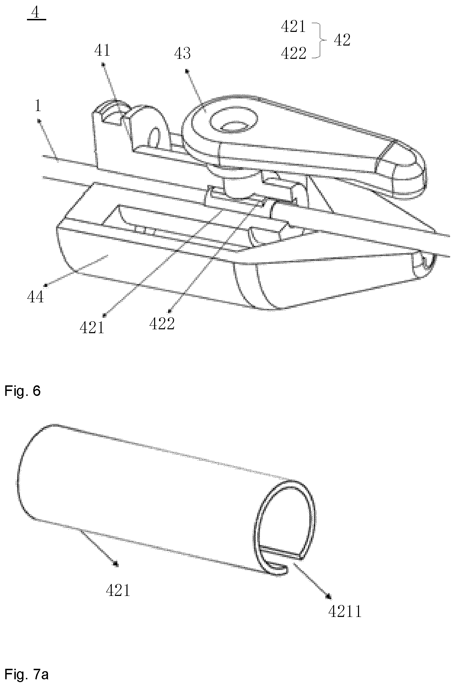

Fig. 6 is a schematic cross-sectional view of a part of the locking handle according to an embodiment of the present invention. As shown in Fig. 6, in order to overcome the above problem, the locking handle 4 according to this embodiment includes a locking shell 44, a locking center piece 41, a locking part 42 and a locking knob 43. The locking shell 44 comprises an internal space in which the locking center piece 41 is fixedly disposed. The locking part 42 is disposed over an outer circumference of the inner catheter 1 within the locking center piece 41.The locking knob 43 extends through a side wall of the locking shell 44, and a part of the locking knob 43 is disposed out of the locking shell 44. The locking part 42 is sandwiched between the locking knob 43 and the inner catheter 1, and the locking knob 43 is adapted to switch the inner catheter 1 between locked and unlocked configurations through changing a radial pressure which applies to the locking part 42.

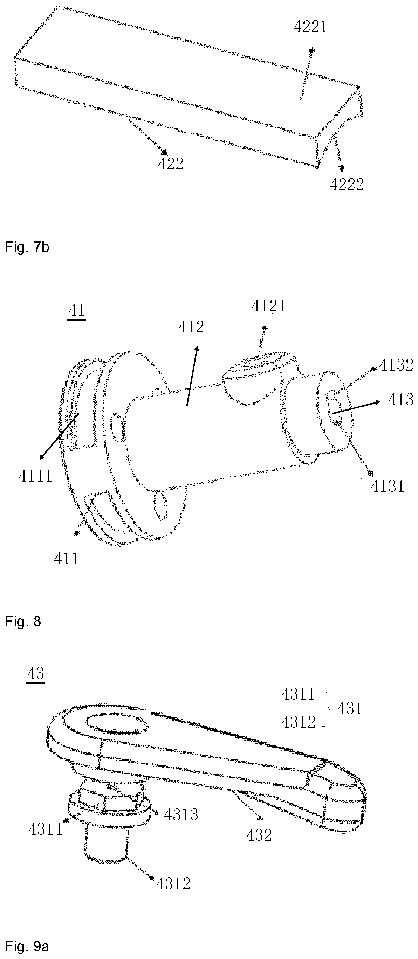

Fig. 7a is a schematic illustration of a collet according to an embodiment of the present invention. Fig. 7b is a schematic illustration of a collet block according to an embodiment of the present invention. As shown in Figs. 7a to 7b, the locking part 42 includes the collet 421 and the collet block 422. The collet 421 is sleeved over the inner catheter 1, and the collet block 422 is disposed on the side of the collet 421 away from the inner catheter 1. That is, the collet 421 is sandwiched between the collet block 422 and the inner catheter 1.

The collet 421 is, for example, a tubular structure, such as a metal tube with a wall thickness ranging from 0.1 mm to 0.8 mm. The collet 421 has an inner diameter greater than an outer diameter of the inner catheter 1. The collet 421 has a slot 4211 axially extending through the side wall of the collet 421 so that the collet 421 has a radial cross-section in the shape of an open ring at any point along its length. When the collet 421 is radially compressed, a circumferential size of the slot 4211 will shrink, and the collet 421 will contract radially inward. As a result, the collet 421 will have a reduced inner diameter that may be smaller the outer diameter of the inner catheter 1. That is, the collet 421 may be compressed so that its inner diameter is smaller than the outer diameter of the inner catheter 1. In this way, the inner catheter 1 can be locked.

The collet block 422 may comprise the shape of a block having an arc-shaped surface 4222 facing the collet 421 and complementary in shape to an outer wall of the collet 421 (i.e., the surface of the collet block 422 facing the collet 421 is an arc-shaped surface 4222 having a diameter that is equal to an outer diameter of the collet 421). A surface of the collet block 422 facing away from the collet 421 may be, for example, a flat surface 4221, which can provide the locking knob 43 with a relative large contact surface. In this way, the locking knob 43 can exert a radial pressure on the large flat surface, which is then transferred to the arc-shaped surface 4222 and dispersed axially and circumferentially over the side wall of the collet 421.

Fig. 8 is a schematic illustration of the locking center piece according to an embodiment of the present invention. As shown in Fig. 8, the locking center piece 41 includes a distal end structure 411 and a proximal end structure 412, which are both cylindrical. The distal end structure 411 has a diameter greater than a diameter of the proximal end structure 412.

The locking center piece 41 comprises an axial bore 413 axially extending therethrough, in which both the inner catheter 1 and the collet 421 are disposed. The inner catheter 1 extends beyond a proximal end of the axial bore 413. The axial bore 413 may be circular, for example. At the proximal end of the axial bore 413, there are provided a mating groove 4132 and a mating locating protrusion 4131, which are arranged circumferentially. The mating groove 4132 communicates with the axial bore 413, and its shape matches the shape of the collet block 422. Thus, the mating groove 4132 may be, for example, a rectangular groove for accommodating the collet block 422. Specifically, arc-shaped surface 4222 of the collet block 422 the faces toward an axis of the axial bore 413 and the flat surface 4221 of the collet block 422 faces away from the axis of the axial bore 413. The mating locating protrusion 4131 may be a cylindrical or elongate stud and configured to be received in the slot 4211 of the collet 421. The mating locating protrusion 4131 has a circumferential length that is smaller than a circumferential length of the slot 4211 when the inner catheter 1 is locked by the collet 421. The mating locating protrusion 4131 is provided to limit circumferential rotation of the collet 421 while not interfering with the radial contraction of the collet 421.

The distal end structure 411 has a side wall provided therein with a first locating groove 4111 configured to secure the distal end structure 411 in the locking shell 44. A second through hole 4121 is provided in an outer circumference of a proximal end portion of the proximal end structure 412, the second through hole 4121 extends through a side wall of the proximal end structure 412 and come into communication with the mating groove 4132. That is, the second through hole 4121 is located external to the mating groove 4132. The mating groove 4132 has an axial length greater than a diameter of the second through hole 4121. An internal thread is provided in the second through hole 4121 for threaded connection with the locking knob 43.

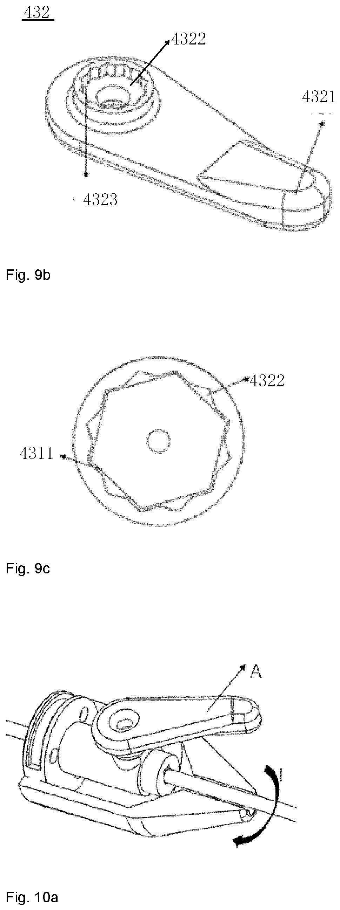

Figs. 9a to 9c are schematic illustrations of the locking knob according to an embodiment of the present invention. As shown in Figs. 9a to 9c, the locking knob 43 includes a connecting part 431 and a dial part 432. The connecting part 431 has a hexagonal boss 4311 and a threaded shaft 4312 provided with an external thread engageable with the internal thread in the second through hole 4121. This enables the connecting part 431 to be threadedly connected within the second through hole 4121. An end portion of the threaded shaft 4312 away from the hexagonal boss 4311 may extend into the mating groove 4132, and by changing a length of the locking knob 43 in the mating groove 4132, a radial pressure on the collet 421 can be adjusted and hence the radial diameter of the collet 421. In this way, the inner catheter 1 can be switched between the locked and unlocked configurations. The connecting part 431 includes a threaded blind hole 4313 in a surface of the hexagonal boss 4311. An internal thread is provided in the threaded blind hole 4313.

The dial part 432 includes a manipulation piece 4321 and a raised annulus 4322 provided on the manipulation piece 4321. The raised annulus 4322 has an inner annular side wall, which is generally circular and provided therein with 12 angularly equidistant second locating grooves 4323, which are V-shaped and outwardly protruded. An angle of each second locating groove 4323 is equal to angle formed between any two adjacent sides of the hexagonal boss 4311 of the connecting part 431. The hexagonal boss 4311 is disposed in the raised annulus 4322, with its corners fitting in respective second locating grooves 4323. Thus, there are 12 possible relative positions of the hexagonal boss 4311 and the second locating grooves 4323, which are spaced apart at an angular interval of 30°. This can ensure that, at an initial position of the locking knob 43 for applying a pressure on the collet block, the manipulation piece 4321 of the dial part 432 is oriented toward a proximal end of the delivery system within an angle range of 30 degrees left to an axis of the catheter. A third through hole is provided at a center of the raised annulus 4322 in the dial part 432, and is coaxial with the threaded blind hole 4313. The dial part 432 and the connecting part 431 are threadedly coupled together by a screw engaged in the third through hole and the threaded blind hole 4313.

The internal spaced provided by the locking shell 44 extends in an axial direction of the locking shell 44. Slots 4211 are formed in both distal and proximal end faces of the locking shell 44. The inner catheter 1 is passed into the locking shell 44 through the distal end slot 4211 and out of the locking shell 44 through the proximal end slot 4211. A first locating stud is provided in the first locating groove 4111 around a distal end of the internal space in order to secure the locking center piece 41 in the locking shell 44. The distal end of the locking shell 44 is coupled to the proximal end of the control shell 36, and the second part 312 of the control center piece 31 is coupled to the distal end structure 411 of the locking center piece 41.

In order to assemble the handle, the locking handle 4 is first secured to a distal end of the control handle 3, and the inner catheter 1 with the collet 421 being disposed thereover is inserted into the axial bore 413 of the locking center piece 41 so that the mating locating protrusion 4131 is located in the slot 4211 of the collet block 422. The collet block 422 is placed in the mating groove 4132 external to the collet 421. Next, the threaded shaft 4312 of the connecting part 431 is screwed into the second through hole 4121 until it is just about to exert a radial pressure on the collet block 422. The locking center piece 41 is fixed within the locking shell 44 by cooperation of the first locating groove 4111 with the first locating stud, and the dial part 432 is placed over the hexagonal boss 4311 so that the hexagonal boss 4311 is received in the raised annulus 4322, and fixed to the connecting part 431 by a screw. The manipulation piece 4321 may be oriented proximally so as to form an angle with the axis of the inner catheter 1. At this time, the inner catheter 1 is in the unlocked configuration (as shown in Fig. 10a), with the manipulation piece 4321 being at a first position A.

A process for switching the inner catheter 1 from the unlocked configuration where the manipulation piece 4321 is at the first position A to the locked configuration begins with pivoting the manipulation piece 4321 in a first direction I to further tighten the locking knob 43 into the mating groove 4132 to exert a radial pressure on the collet block 422. The radial pressure is then dispersed to the collet 421, reducing the inner diameter of the collet 421 and thereby causing it to lock the inner catheter 1. In this way, the inner catheter 1 is brought into the locked configuration, where the inner catheter 1 is prevented from axial displacement (as shown in Fig. 10b) and the dial part 432 is at a second position B. The inner catheter 1 can be switched back to the unlocked configuration by pivoting the dial part 432 in the direction opposite to the first direction I and returning the manipulation piece 4321 to the first position A. As a result, the locking knob 43 moves away from the mating groove 4132, reducing the radial pressure on the collet block 422 to zero or nearly zero. This results in expansion of the inner diameter of the collet 421 to the original size. As the radial pressure has disappeared, axial displacement of the inner catheter 1 becomes again possible.

According to embodiments of the present invention, between the control handle 3 and the locking handle 4, a seal is desirably formed between the flexible catheter 2 and the inner catheter 1. The present invention is not limited to any particular form or structure of the seal.

Use of the implant delivery system will be exemplified below by way of use of the control handle 3 in a transfemoral aortic valve replacement procedure. At first, an implant (e.g., valve) is loaded onto a balloon disposed at the distal end of the inner catheter 1, then enable a proximal end of the implant to be located near the distal end of the flexible catheter 2. Subsequently, the dial part 432 is pivoted to apply a radial pressure to the collet 421 through the collet block 422 to reduce the inner diameter of the collet 421 and thus bring the inner catheter 1 into the locked configuration. Upon the valve being advanced to the aortic arch through the femoral artery, while the inner catheter 1 and the flexible catheter 2 are being further pushed forward, the control handwheel 33 is rotated to pull the control wire via the slider 32 to control bending of the catheter and the flexible catheter 2. After the valve reaches the target site, the dial part 432 is pivoted to eliminate the radial pressure from the collet block 422, switching the inner catheter 1 to the unlocked configuration. With the inner catheter 1 being held stationary, the flexible catheter 2 is retracted, and the manipulation piece 4321 is pivoted to switch the inner catheter 1 back to the locked configuration. After valve placement and pacing have been achieved, the delivery system is withdrawn (as shown in Fig. 1c).

With the implant delivery handle, delivery system and method of operation provided in the present invention, a catheter can be switched between locked and unlocked configurations by compressing or uncompressing a slotted collet disposed thereover and thereby changing its inner diameter. This allows a locking action to be accomplished horizontally and thereby distinctly distinguishes it from a bending control action taken by rotating a rotary wheel, effectively preventing a surgeon from being confused between the two actions. Instead of locking a catheter by circumferentially rotating a rotary wheel as is conventional, the present invention enables locking and releasing of a catheter by compressing and uncompressing a slotted metal collet. Bending control can be achieved with a simple, easy-to-assemble structure. Specifically, the handle can be rotated clockwise to deliver a bending control function or counterclockwise to disable the bending control function by a single hand with little physical effort. Designing the flexible catheter 2 and the balloon catheter as separate catheters enables accurate bending control during advancement through the aortic arch through keeping the implant in the vicinity of the flexible catheter 2. Further, during balloon pacing, the flexible catheter 2 can be retracted away to avoid it from affecting the pacing

It is to be noted that, as used herein, the terms "first" and "second" are only meant to distinguish various components, elements, steps, etc. from each other rather than indicate logical or sequential orderings thereof, unless otherwise indicated or specified.

It is to be understood that while the invention has been described above with reference to preferred embodiments thereof, it is not limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. An implant delivery handle, comprising a locking handle disposed along an axial direction of a catheter assembly, wherein the locking handle comprises a locking part and a locking knob, wherein the locking part is sleeved over an inner catheter in the catheter assembly and is sandwiched between the locking knob and the inner catheter, and wherein the locking knob is configured to pivot about an axis of the inner catheter to apply a radial pressure to the inner catheter through the locking part, thereby switching the inner catheter between an unlocked configuration and a locked configuration,
wherein the locking part comprises a collet sleeved over the inner catheter, wherein the locking knob is in abutment against the collet, wherein the collet has an inner diameter greater than an outer diameter of the inner catheter and has a slot axially extending therethrough, wherein in case of the collet being subject to a radial pressure, a circumferential length of the slot decreases, resulting in a reduced inner diameter of the collet, wherein the reduced inner diameter of the collet is smaller than the outer diameter of the inner catheter, and wherein the collet locks the inner catheter through contraction of the collet.

2. The implant delivery handle of claim 1, comprising a control handle disposed along the axial direction of the catheter assembly, wherein the control handle is disposed at a distal end of the locking handle, and wherein the control handle is coupled to a flexible catheter in the catheter assembly,
wherein the control handle comprises a control handwheel coupled to a control wire on the flexible catheter, and wherein the control handwheel is able to rotate to exert a force on the control wire to provide a bending control to the catheter assembly.

3. The implant delivery handle of claim 1, wherein the locking knob further comprises a collet block disposed over an outer surface of the collet, wherein the locking knob is configured to abut against the collet via the collet block, wherein a surface of the collet block facing the collet matches the outer surface of the collet in shape, and wherein a surface of the collet block facing the locking knob is flat.

4. The implant delivery handle of claim 3, wherein the locking handle further comprises a locking center piece and a locking shell, wherein the locking center piece is secured within the locking shell, wherein the locking knob extends through a sidewall of the locking shell and is threadedly coupled to a sidewall of the locking center piece,
wherein the locking center piece comprises an axial bore axially extending therethrough, wherein the inner catheter and the collet are disposed in the axial bore, and wherein a proximal end of the axial bore is provided with a mating groove and a mating locating protrusion that are circumferentially arranged, wherein the mating groove is configured for fitting of the collet block and the mating locating protrusion is received in the slot.

5. The implant delivery handle of claim 4, wherein the locking knob comprises a connecting part and a dial part, wherein the connecting part extends through the sidewall of the locking shell, and is threadedly coupled to the sidewall of the locking center piece and is configured to abut against the collet block,
wherein the dial part comprises a manipulation piece sleeved over an end portion of the connecting part away from the collet block and threadedly fastened to the connecting part, wherein the manipulation piece is disposed facing a proximal end of the implant delivery handle and is configured to drive, at a proximal end of the locking handle, the connecting part to pivot about the axis of the inner catheter.

6. The implant delivery handle of claim 1, wherein the control handle further comprises:
a control shell comprising an inner lumen, wherein the inner lumen comprises a distal first portion and a proximal second portion, wherein an inner diameter of the first portion is smaller than an inner diameter of the second portion;
a control center piece, inserted in the inner lumen, wherein the control handwheel is sleeved over the control center piece;
a proximal snap part disposed in the first portion;
a distal snap part disposed in the first portion and located at a distal end of the proximal snap part, wherein the distal snap part has an outer diameter greater than an inner diameter of the first portion; and
a slider threadedly coupled to the control handwheel and located at a proximal end of the proximal snap part, wherein the slider is able to move axially between the proximal snap part and a proximal end portion of the control handwheel.

7. The implant delivery handle of claim 6, wherein the control center piece comprises a tubular first part and a tubular second part sleeved over a proximal end portion of the first part, wherein a flexible catheter is secured in the first part, and wherein the control handwheel is sleeved over the first part,
wherein the first part is provided in an outer circumference thereof with a proximal snap slot and a distal snap slot, which are axially spaced apart from each other, wherein the proximal snap slot is provided at a distal end of the control handwheel and is configured to secure the proximal snap part, and wherein the distal snap slot is configured to secure the distal snap part,
wherein the first part is further provided in the outer circumference thereof with a wire slot, wherein the wire slot extends through a sidewall of the first part and has a same axial direction as the first part, wherein the wire slot is located between a distal end face of the first part and the proximal snap slot, and wherein a control wire in the flexible catheter leads out the first part through the wire slot and is tied to the slider.

8. The implant delivery handle of claim 6, wherein the control handwheel comprises a proximal portion and a distal portion which are joined to each other, wherein the proximal portion has an outer diameter greater than an outer diameter of the distal portion, wherein a distal end face of the proximal portion comprises a receptacle having a same axial direction as the first part, wherein a diameter of a side wall of the receptacle proximal to an axis is equal to the outer diameter of the distal portion,
wherein the slider is threadedly coupled to an outer wall of the distal portion and to the receptacle, and wherein the slider is able to move axially on the control handwheel between the proximal snap part and a bottom surface of the receptacle.

9. An implant delivery system, comprising the implant delivery handle of any one of claims 1 to 8, the catheter assembly and a Y-shaped catheter holder, wherein the Y-shaped catheter holder is disposed at a proximal end of the implant delivery handle, wherein the catheter assembly comprises the inner catheter and a flexible catheter sleeved over the inner catheter, wherein a proximal end of the inner catheter passes through the implant delivery handle and is secured to the Y-shaped catheter holder, wherein the flexible catheter is secured to the control handle of the implant delivery handle, wherein the control handle is configured to enable the flexible catheter to provide a bending control, and wherein the locking handle of the implant delivery handle is configured to switch the inner catheter between the locked and unlocked configurations against axial displacement of the inner catheter.

10. A method of operating the implant delivery system of claim 9, comprising:
rotating the control handwheel to cause a distal end of the control wire to bend as a result of a force exerted by the slider on the control wire increasing, or to cause the distal end of the control wire to return to an original configuration as a result of a force exerted by the slider on the control wire decreasing; and
pivoting the dial part to a first position where the locking part does not exert a radial pressure on the collet and the inner catheter is in the unlocked configuration in which the inner catheter is capable of an axial displacement; pivoting the dial part to a second position where the locking part exerts a radial pressure on the collet to reduce an inner diameter of the collet, and the inner catheter is in the locked configuration in which the inner catheter is incapable of an axial displacement.
